# EUROPEAN PATENT SPECIFICATION

(11) **EP 0 600 864 B1**
(45) Date of publication and mention of the grant of the patent: **04.09.1996**
(21) Application number: 91910303.6
(22) Date of filing: 23.05.1991
(51) Int. Cl.: A61M 27/00, A61B 17/34

(54) **DEVICE FOR APPLYING A DRAIN**
VORRICHTUNG ZUM ANBRINGEN EINES DRAINS
DISPOSITIF SERVANT A APPLIQUER UN DRAIN

(30) Priority: 23.05.1990 SE 9001857
(43) Date of publication of application: 15.06.1994
(73) Proprietor: ATOS MEDICAL AB, S-242 93 Hörby (SE)
(72) Inventor: PERSSON, Jan-Ove, S-243 95 Höör (SE); LEJDEBORN, Lars, S-162 24 Vällingby (SE); BERG, Olle, S-181 47 Lidingö (SE)
(74) Representative: Ström, Tore
(86) International application number: PCT/SE91/00371
(87) International publication number: WO 91/17787

(56) References cited:
- DE-A- 1 791 178
- SE-B- 450 996
- US-A- 3 659 610

## Description

The invention relates to a device for applying a drain to an antrum substantially surrounded by bone, such as the maxillary sinus, comprising a catheter, an elongated needle and means for displacing the needle between a proximal retracted position and a distal end position for penetration of the bone wall by the needle in said distal end position thereof.

A device of this kind is disclosed in US-A-3,659,610.

In the area of both human and veterinary medicine it is often desirable to get access to an antrum, for instance in inflammatory conditions when pus, blood or secretion is to be discharged or in other conditions when gases or liquids are to be evacuated. An example in human medicine is infection in the maxillary sinus including pus therein, accumulation of blood or air in the thoracic cavity after a trauma, urinary retention disabling depletion of the bladder, or conditions including accumulation of blood within or outside the hard meninx. Also sampling from antrums are customary, for instance bone marrow sampling from medullary cavities.

Infection of maxillary sinus including pus implies specific problems. As a result of swelling of the mucous membrane in nose and maxillary sinus at an upper respiratory infection the normal outlets of the maxillary sinus are closed. The air normally existing in the maxillary sinus will be resorbed and the partial pressure decreases. Normally, already at this point the patient experiences a feeling of weight or pressure from the affected maxillary sinus. If the process continues the transport by the cilia is stopped and secretion of the mucous membrane is accumulated. In such an environement the immunulogical defence against bacteria is deteriorated, and a bacterial infection with an accumulation of pus could arise. Because of the size and the anatomic structure with an outlet passage in a high position the maxillary sinus is extremely exposed to bacterial infections. There could also be a spreading of infections to the maxillary sinus from infected teeth of the upper jaw. The treatment comprises puncture of the maxillary sinus by inserting a needle into the nasal cavity and engaging the wall of the maxillary sinus. Then the needle is forced through the bone wall into the maxillary sinus. In this way material accumulated in the maxillary sinus can be sucked out for diagnosis, and then the maxillary sinus can be rinsed with a sodium cloride solution. However, it is difficult to puncture the maxillary sinus in this way. A careful anaesthesia is necessary and the doctor in charge has to be experienced. In spite of this also experienced otorhinolaryngologists fail when puncturing. A major cause of the problem is that the needle must be pressed through the bone in an oblique angle. A low space anatomy or a thick bone structure of the maxillary sinus in many cases inhibits a puncture. Instead the needle will slide in the mocous membrane and cause a bleeding. Sometimes the maxillary sinus is reached only after applying a very heavy force on the needle and in such a case there is a risk that the needle will move forward through the rear wall of the maxillary sinus or into the eyeball; such complications are well documented. However, as the swelling of the mocous membrane, the closed outlet and a reduced transporting ability of the cilia will remain for a longer period of time (days or weeks), even if the treatment is favourable, it is normally necessary to repeat the treatment several times before a complete healing of the infection is achieved. Each treatment requires a new puncture resulting in further discomfort to the patient and requiring comparatively large costs of treatment of a specialist. In practice this means that repeated antral washouts are performed only every second or every third day, even though from a medical point of view it would be more appropriate and lead to a faster healing if the antral washouts were made at least on a daily basis. Thus, it is desirable to decrease demands of repeated punctures in each case but nevertheless have the possibility of performing an antral washout as often as possible.

There have been attempts to decrease the number of repeated punctures, for instance by inserting a comparatively rigid catheter in the needle. When pressed into the maxillary sinus in a normal way the needle has been retracted. By a special shape of the catheter which either has the shape of a spiral or is provided with flanges, the catheter has been prevented from falling out of the maxillary sinus and it has been possible to perform antral washouts repeatedly without the necessity of repeated punctures. However, the methods have not become as popular as could have been expected. The reason for this is that none of the methods overcomes the difficulties and risks of forcing the needle into the maxillary sinus; instead a larger force is required to insert the needle and the catheter. Furthermore, the function of the spiral-preshaped catheter has been unsafe and the catheter has often come out of the maxillary sinus. Both of said types of catheters have been manufactured of rigid plastics to make possible the insertion which means that they have been uncomfortable to the patients and caused problems and pain in the opening of the nose.

An object of the present invention is to overcome essentially the drawbacks and the problems mentioned above.

The object has been accomplished by providing a device of the kind referred to having the characterising features of claim 1.

DE-A-1,791,178 discloses a device having a needle which is inserted into a drain. A further object of the invention is to make possible administration of medicines through the device.

The invention will now be described in more detail with reference to the accompanying drawings, in which,
- FIG. 1: is a longitudinal sectional view of a drain for draining the maxillary sinus,
- FIG. 2: is an enlarged longitudinal sectional view of a portion of the drain of FIG. 1 having a needle extending therethrough,
- FIG. 3: is a sideview of the needle of FIG. 2,
- FIG. 4: is a longitudinal sectional view of a device according to the invention for applying the drain of FIGS. 1-3,
- FIG. 5: is a sideview of an alternative embodiment of the drain, and
- FIG. 6: are two sideviews from different directions of still another embodiment of the drain.

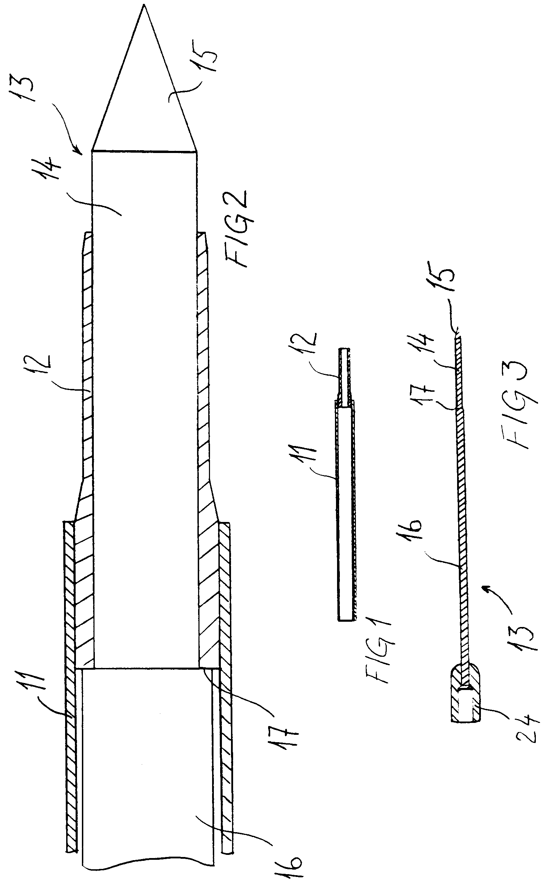

The drain for maxillary sinus according to FIG. 1 comprises a hose or catheter 11 and a tube 12 connected thereto. Said catheter 11 is made of a soft and flexible material so as to extend, after applying said drain, through the nose without any major inconveniences to the patient and to be available from the opening of the nose. A suitable material for said catheter is rubber or thermoplastic elastomer preferably silicone rubber and vinyl chloride plastic. Said tube 12 is made of a rigid and hard material and is provided with thin walls so as to be inserted easily together with a needle through the wall of maxillary sinus. Materials, such as metal, for instance titanium or steel, or rigid plastic can be used in said tube 12. In a preferred embodiment said tube and said catheter are fixedly interconnected, preferably by gluing, and said catheter surrounds a section of said tube. From the description below it is clear that in the described embodiment the tube will engage a shoulder of a needle.

FIG. 2 shows a state in which said catheter 11 and said tube 12 have been passed onto a needle 13, said needle comprising a needle point 15, a point section 14 and a base section 16. Between said point section 14 and said base section 16 there is provided a stop formed as a radially extending shoulder 17. Said tube 12 engages said shoulder 17 when put on the needle 13 from the needlepoint thereof thereby being prevented from further displacement backwards. The end of said tube 12 directed towards the needle point is bevelled so as not to be restricting when said needle together with catheter and tube is inserted through the bonewall between the nasal cavity and the maxillary sinus.

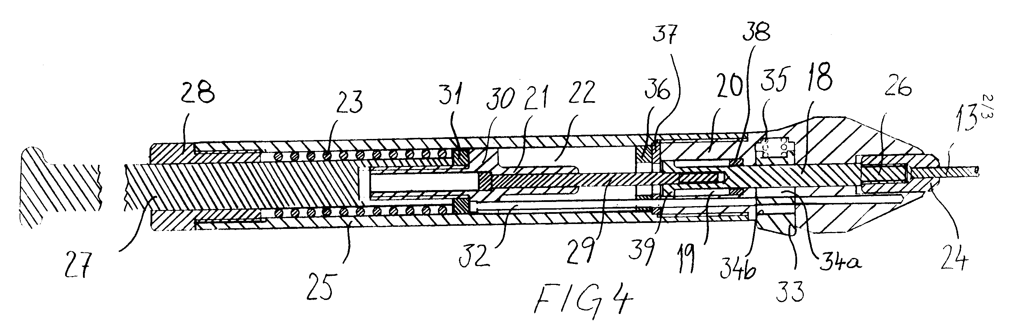

FIG. 3 shows the entire needle 13. The end of said needle opposite to the needle point comprises an internally threaded socket 24. Said socket is screwed together with the device described below before the drain is applied. In the preferred embodiment said needle and said socket are made of a material facilitating a reuse after disinfection, for instance steel, but it is also possible to provide needle and socket as expendable products.

To overcome essentially problems during penetration of the bonewall into the maxillary sinus and the insertion of the drain therein a device according to the invention is provided. Said device is shown in FIG. 4 and comprises a cylindrical cover 25 having a head 20 and an end section 28 enclosing several axially arranged hollow spaces. In a first hollow space 19 in said head 20 a needle holder 18 having a threaded front section 26 and a needle 13 detachably connected to said needle holder 18 are arranged axially displaceable. A restriction in displacing the needle holder in the backward direction is provided by a washer 37 and in the forward direction by an impact damper 38 arranged in said first hollow space, said damper being engaged by an exterior flange 39 of said needle holder. A front section 26 of said needle holder is threadedly connected to said socket 24, and a rear section of said needle holder is threadedly connected to one end of a return pin 29, a second end thereof being received axially displaceable within a hammer 21 arranged axially displaceable in said cover.

Said hammer 21 can be moved in a second, elongated and axially extending hollow space 22, the end thereof opposite to said head being closed by an annular damper 36. The end of the return pin received in said hammer comprises an exterior flange engaging an interior shoulder of said hammer when said hammer is moved in the backward direction. Said hammer is connected by external threads in the end thereof opposite to said head to a partly retractable cocking lever 27. Said cocking lever extends through said end section 28 which is screwed into said cylindrical cover 25. Said hammer is provided with an external flange 30. A stop washer 31 is provided engaging said flange 30, and between said stop washer 31 and said end section a coil spring 23 is fixed, said spring being depressed when said cocking lever is moved backwardly from said head. A spacer 32 is provided extending from said hammer towards said head to lock said cocking lever and hammer in a state of tension. Said spacer 32 extends through and is guided in an axial recess in said head and engages an elongated releasing pin 33 that is radially displaceable in a radial recess in said head. Said releasing pin 33 is formed with a first through opening 34a extending in the transverse direction of said releasing pin, the needle holder 18 extending through said recess, and also a smaller recess 34b extending in the same direction through which the releasing pin 33 moves when it is disposed in an inner position in said radially extending recess. A spring 35 is provided in the bottom of said radially extending recess, said spring forcing said releasing pin outwardly towards an outer position in which said releasing pin is prevented from a further movement forwardly.

Said hammer is cylindrical and is provided with a thin front section directed towards said head, the outer diameter of said section being smaller than the inner diameter of said annular damper 36 and said washer 37. Thereby said hammer can be moved through said damper 36 and said washer 37.

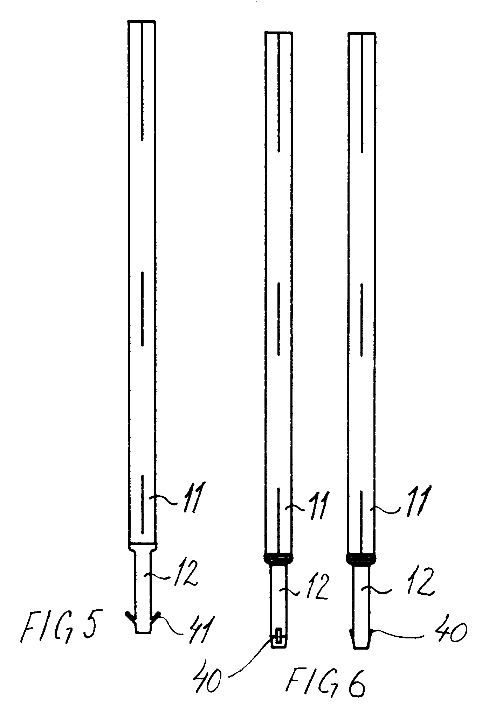

FIG. 5 and FIG. 6 show two developments of said drain. Said tube 12 is provided with elements to hamper an incidental retraction of the drain. Said tube 12 of said drain according to FIG. 5 is provided with barbs 41 in the free end thereof. Such an embodiment is intended first of all for use for patients having a bad wall of the maxillary sinus, for instance patients previously exposed to medical surgery of said wall or patients that have been punctured frequently in a conventional manner. The barbs are very thin, approximately 0,25 mm, and thereby they are folded during penetration and refolded when retracted.

In the embodiment according to FIG. 6 said tube 12 of the drain is provided with extending wedge shaped hooks 40, the thinner part thereof being disposed in the insertion direction of said tube.

The function of the device described above will now be described in more detail. A needle 13 is screwed onto said threaded section 26 of said needle holder 18, and then said cocking lever 27 is pulled backwards against said spring 23. Said return pin 29 will also move backwards, thereby bringing also said needle holder backwards. When the spacer 32, while moving together with said hammer 21, has passed through said recess 34b in said releasing pin 33 said spring 23 will bring said releasing pin to an upper position in which said spacer is prevented from further movement in the forward direction. Now the device is in a ready state shown in FIG. 4. The drain is put onto said needle 13, which is inserted into the nose to make the needlepoint engage the bone of the maxillary sinus. In advance a local anasthetic has been given in the nose in a suitable way, for instance by an anasthetic spray. Said releasing pin 33 is depressed to allow said spacer 32 pass through said recess 34b. Then the hammer 21 is accellerated by the force of said spring 23 and moves through said damper 36 and said washer 37 and hits the rear section of said needleholder 18, which together with said needle is pushed very rapidly forwards to make said needle penetrate the bone wall and the front section of the drain pass through said bonewall. During the forward movement of said hammer said return pin 29 is not directly affected by said hammer. The movement in the forward direction of the needle holder 18 is slowed down when the flange 39 of said needle holder engages said impact damper 38 and when said flange 30 of said needle 21 engages said damper 36. The length of stroke of said needle is determined by the distance between said washer 37 and said impact damper 38 and said distance is chosen from the anatomic conditions of nose. Then the device including the needle is removed from the nose while leaving said drain. Then repeated washouts and tests can be made without any further penetrations being required because the flexible catheter of the drain is easily available from the opening of the nose.

The very good action of the device is mostly based on the fast pushing, the well determined length of stroke, and the needle being slowed down in a controlled way. Other embodiments of the device giving the same function can be provided within the scope of the invention.

The embodiment of said releasing device shown, can be varied in many ways within the scope of the invention, for instance by using releasing elements from the arms industry. The stop 17 consisting in the shown embodiment of a shoulder can instead in other embodiments comprise one or several radially extending pins or a ring mounted in a groove. Also other embodiments of a stop for restricting an axial displacement of the tube and catheter when put on the needle are possible.

The drain is also to a high degree applyable when administrating medicine or other substances to the antrum. To facilitate administration of medicine through the drain in different areas said catheter in one embodiment is provided with a fixed adapter in the free end thereof, said adapter being intended for receiving a cannula or the like. In an alternative embodiment the free end of said catheter is formed so as to be detachably connected to different types of adapters. The catheter is connected to syringes, dosage aggregates, suction devices or the like.

For specific types of medicine or during specific circumstances it is appropriate to administer medicine into the antrum through a hose inserted through the catheter, said hose having a smaller diameter than said catheter. Such a method is appropriate especially when the medicine is intended for a certain part of the antrum.

For patients requiring a continuous ventilation of an antrum, for instance the maxillary sinus, the drain is provided with a shortened catheter or the catheter is excluded.

In the last described embodiment the end of the tube opening into said antrum preferably is provided with an exterior flange so as to reduce the risk of said tube unintentionally falling out. Also when using the drain according to the first described method it is under certain circumstances appropriate to provide said tube with such a flange.

## Claims

1. Device for applying a drain to an antrum substantially surrounded by bone, such as maxillary sinus, comprising a catheter (11), an elongated needle (13), and means for displacing the needle between a proximal retracted position and a distal end position for penetration of the bone wall by the needle in said distal end position thereof, **characterised** in that the catheter (11) is made of soft and flexible material and at its distal end is connected to a tube (12), that the needle (13) is slidable through the catheter (11) and the tube (12) which is made of a rigid and hard material so as to be insertable together with the needle, said needle engaging said tube by means of a shoulder, and that said means for displacing the needle comprises a hammer (21) displaceable between a proximal end position and a distal end position, the needle in the proximal retracted position thereof being positioned at a predetermined distance from said proximal end position of the hammer (21) so as to be moved a predetermined distance by a stroke of the hammer at displacement of the hammer (21) to the distal end position thereof, said needle carrying along the tube and the catheter at said shoulder.

2. Device according to claim 1, **characterised** in that the needle (13) comprises a distal end section (14) terminating in a needle point (15) the tube (12) being passed onto the end section to be located between the needle point and an element (17) on the needle engaging the distal end of the tube.

3. Device according to claim 1 or 2, **characterised** in that the element (17) comprises an annular shoulder on the needle.

4. Device according to any of claims 1 to 3, **characterised** in that the tube (12) at the free end thereof is provided with means (40, 41) projecting from the surface thereof for preventing incidental retraction of the drain from the antrum.

5. Device according to claim 4, **characterised** in that said means comprises wedges (40).

6. Device according to claim 4, **characterised** in that said means comprises barbs (41).

## Patentansprüche

1. Vorrichtung zum Anbringen eines Drains an einem im wesentlichen durch Knochen umgebenen Antrum, beispielsweise Sinus maxillaris, mit einem Katheter (11), einer langgestreckten Nadel (13) und mit einer Einrichtung zum Verschieben der Nadel zwischen einer proximalen zurückgezogenen Position und einer distalen Endposition zum Durchdringen der Knochenwand durch die Nadel in ihrer distalen Endposition, **dadurch gekennzeichnet**, daß der Katheter (11) aus einem weichen und flexiblen Material hergestellt ist und an seinem distalen Ende mit einer Leitung (12) verbunden ist, daß die Nadel (13) durch den Katheter (11) und die Leitung (12) verschiebbar ist, wobei die Leitung (12) aus einem starren und harten Material hergestellt ist, um gemeinsam mit der Nadel einführbar zu sein, wobei die Nadel an der Leitung mittels einer Schulter angreift, und daß die genannte Einrichtung zum Verschieben der Nadel einen Hammer (21) aufweist, der zwischen einer proximalen Endposition und einer distalen Endposition verschiebbar ist, wobei die Nadel in ihrer proximalen zurückgezogenen Position mit einer vorgegebenen Distanz von der genannten proximalen Endposition des Hammers (21) positioniert ist, so daß die Nadel um eine vorgegebene Distanz durch einen Hub des Hammers bei Verschiebung des Hammers (21) zu seiner distalen Endposition hin bewegt wird, und wobei die genannte Nadel die Leitung und den Katheter an der genannten Schulter mitnimmt.

2. Vorrichtung gemäß Anspruch 1, **dadurch gekennzeichnet**, daß die Nadel (13) einen distalen Endabschnitt (14) aufweist, der in einer Nadelspitze (15) endet, wobei die Leitung (12) auf den Endabschnitt aufgebracht ist, um zwischen der Nadelspitze und einem Element (17) an der Nadel angeordnet zu werden, welches an dem distalen Ende der Leitung angreift.

3. Vorrichtung gemäß Anspruch 1 oder 2, **dadurch gekennzeichnet**, daß das Element (17) eine ringförmige Schulter an der Nadel aufweist.

4. Vorrichtung gemäß einem der Ansprüche 1 bis 3, **dadurch gekennzeichnet**, daß die Leitung (12) an ihrem freien Ende mit Mitteln (40, 41) versehen ist, die von der Oberfläche der Leitung vorspringen, um ein zufälliges Zurückziehen des Drains von dem Antrum zu verhindern.

5. Vorrichtung gemäß Anspruch 4, **dadurch gekennzeichnet**, daß die genannten Mittel Keile (40) aufweisen.

6. Vorrichtung gemäß Anspruch 4, **dadurch gekennzeichnet**, daß die genannten Mittel Widerhaken (41) aufweisen.

## Revendications

1. Dispositif pour appliquer un drain sur un sinus pratiquement entouré par un os, tel que le sinus maxillaire, comprenant un cathéter (11), une aiguille allongée (13) et un moyen pour déplacer l'aiguille entre une position rétractée proximale et une position d'extrémité distale, pour la pénétration de la paroi de l'os par l'aiguille dans sa dite position d'extrémité distale, caractérisé en ce que le cathéter (11) est constitué d'un matériau souple et flexible, et son extrémité distale est connectée à un tube (12), en ce que l'aiguille (13) est coulissante dans le cathéter (11) et le tube (12), qui est constitué en un matériau rigide et dur de manière à pouvoir être inséré conjointement avec l'aiguille, ladite aiguille s'engageant contre ledit tube au moyen d'un épaulement, et en ce que ledit moyen pour déplacer l'aiguille comprend un marteau (21) déplaçable entre une position d'extrémité proximale et une position d'extrémité distale, l'aiguille se trouvant dans sa position réfractée proximale étant disposée à une distance prédéterminée de ladite position d'extrémité proximale du marteau (21), de manière à être déplacée à une distance prédéterminée par une course du marteau, selon le déplacement du marteau (21) vers sa position d'extrémité distale, ladite aiguille portant le long du tube et du cathéter, au niveau dudit épaulement.

2. Dispositif selon la revendication 1, caractérisé en ce que l'aiguille (13) comprend une section d'extrémité distale (14) se terminant par une pointe d'aiguille (15), le tube (12) étant passé sur la section d'extrémité, pour se trouver entre la pointe d'aiguille et un élément (17) se trouvant sur l'aiguille, s'engageant contre l'extrémité distale du tube.

3. Dispositif selon la revendication 1 ou 2, caractérisé en ce que l'élément (17) comprend un élément annulaire réalisé sur l'aiguille.

4. Dispositif selon l'une quelconque des revendications 1 à 3, caractérisé en ce que le tube (12), au niveau de son extrémité libre, est pourvu de moyens (40, 41) faisant saillie depuis sa surface, pour empêcher toute réfraction involontaire du drain depuis le sinus.

5. Dispositif selon la revendication 4, caractérisé en ce que ledit moyen comprend des angles (40).

6. Dispositif selon la revendication 4, caractérisé en ce que ledit moyen comprend des barbes (41).
